# EUROPEAN PATENT APPLICATION

(11) **EP 4 641 582 A1**
(43) Date of publication of application: **29.10.2025**
(21) Application number: 23903949.8
(22) Date of filing: 12.12.2023
(51) Int. Cl.: G16H 40/20, G16H 40/63, G16H 10/60, G16H 20/10, G16H 70/40, G06K 19/06, G16H 80/00, G16H 10/20, G06Q 10/08

(54) **VACCINE MANAGEMENT SYSTEM FOR PREVENTING VACCINATION ACCIDENT DUE TO INCORRECT VACCINATION AND MISREGISTRATION OF VACCINE, AND VACCINATION SERVICE PROVISION METHOD PERFORMED BY MEANS OF VACCINE MANAGEMENT SYSTEM**

(30) Priority: 13.12.2022 KR 20220173637; 13.10.2023 KR 20230136969; 16.10.2023 KR 20230137503
(71) Applicant: Real Time Medi Check Corp., Wonju-si, Gangwon-do 26460 (KR)
(72) Inventor: KIM, Hee, Incheon 22003 (KR); LIM, Jae Joon, Incheon 22003 (KR)
(74) Representative: Grünecker Patent- und Rechtsanwälte PartG mbB
(86) International application number: PCT/KR2023/020388
(87) International publication number: WO 2024/128746

(57) **Abstract**

There are disclosed system and method for preventing vaccination accidents due to misvaccination and misregistration of vaccines. The system comprises: an on-site vaccination information recognition device for recognizing a subject identification code of a subject to be vaccinated and information on vaccine for vaccination printed on a syringe or vial; and an on-site terminal for displaying check information that must be confirmed for vaccination of the subject to be vaccinated based on the subject identification code and information on vaccine for vaccination transmitted from the on-site vaccination information recognition device, on a screen. The screen of the on-site terminal includes: a first display area for displaying the identity information of the subject to be vaccinated corresponding to the subject identification code of the check information; and a second display area for displaying the vaccination information corresponding to the information on vaccine for vaccination of the check information.

## Description

### [Technical Field]

The Examples of the present invention relate to a vaccine management system for preventing vaccination accidents due to misvaccination and misregistration of vaccines, and a method for providing a vaccination service performed in the vaccine management system.

### [Background Art]

Vaccines are used to prevent or minimize damage from pathogens when a human body is infected with them by injecting pathogens, and the like that have been artificially inactivated or attenuated into the body before infection with the pathogens and activating the body's immune system.

In general, vaccination services are provided in a manner in which vaccines produced by manufacturers are stocked and registered at medical institutions, and medical staff administers the registered vaccines to subjects to be vaccinated who visit medical institutions.

In these vaccination services, vaccination accidents such as misvaccination and misregistration may occur.Misvaccination is a vaccination accident in which a subject to be vaccinated is vaccinated with a different vaccine than the one intended, and misregistration is a vaccination accident in which the drug name, lot number, expiration date, and the like of the vaccine are incorrectly registered during the vaccine registration process, or a subject to be vaccinated is incorrectly registered as having been vaccinated with vaccine B when they were vaccinated with vaccine A.

Therefore, there is a need for technology to prevent vaccination accidents such as misvaccination and misregistration in advance during the process of providing vaccination services.

### [Disclosure]

### [Technical Problem]

It is an object of the present invention to provide a vaccine management system for preventing vaccination accidents due to misvaccination and misregistration of vaccines, and a method for providing a vaccination service performed in the vaccine management system.

It is another object of the present invention to provide a vaccine management system for preventing vaccination accidents due to misvaccination and misregistration by confirming with a subject to be vaccinated and medical staff whether the registered vaccine matches the vaccine to be vaccinated before vaccination, and a method for providing a vaccination service performed in the vaccine management system.

It is yet another object of the present invention to provide a vaccine management system for providing notification of the expiration date of a vaccine, and a method for providing a vaccination service performed in the vaccine management system.

The objects of the present invention are not limited to the above-mentioned objects, and other objects and advantages of the present invention not mentioned may be understood by the following description and will be more clearly understood by the embodiments of the present invention. In addition, it will be readily apparent that the objects and advantages of the present invention may be realized by the means and combinations thereof indicated in the claims.

### [Technical Solution]

The vaccine management system according to one embodiment of the present invention comprises: an on-site vaccination information recognition device for recognizing a subject identification code of a subject to be vaccinated and information on vaccine for vaccination printed on a syringe or vial; and an on-site terminal for displaying check information that must be confirmed for vaccination of the subject to be vaccinated based on the subject identification code and information on vaccine for vaccination transmitted from the on-site vaccination information recognition device on a screen.In this case, the screen of the on-site terminal comprises: a first display area for displaying the identity information of the subject to be vaccinated corresponding to the subject identification code of the check information; and a second display area for displaying the vaccination information corresponding to the information on vaccine for vaccination of the check information.

The method for providing a vaccination service according to one embodiment of the present invention is a method for providing a vaccination service performed in a vaccine management system comprising an on-site vaccination information recognition device and an on-site terminal connected to communication, the method comprising the steps of: recognizing a subject identification code of a subject to be vaccinated and information on vaccine for vaccination printed on a syringe or vial by the on-site vaccination information recognition device and transmitting them to the on-site terminal; and displaying check information that must be confirmed for vaccination of the subject to be vaccinated based on the subject identification code and information on vaccine for vaccination on a screen of the on-site terminal.In this case, the screen of the on-site terminal comprises: a first display area for displaying the identity information of the subject to be vaccinated corresponding to the subject identification code of the check information; and a second display area for displaying the vaccination information corresponding to the information on vaccine for vaccination of the check information.

### [Advantageous Effects]

According to the present invention, vaccination accidents due to misvaccination and misregistration of a vaccine may be prevented.

In addition, according to the present invention, vaccination accidents due to misvaccination and misregistration may be prevented by confirming with a subject to be vaccinated and medical staff whether the registered vaccine matches the vaccine to be vaccinated before vaccination.

In addition, according to the present invention, a situation in which a subject to be vaccinated is vaccinated with a vaccine whose expiration date has expired may be prevented by providing notification of the expiration date of the vaccine.

In addition, it should be understood that the effects of the present invention are not limited to the effects described above, but include all effects that can be inferred from the configuration of the present invention described in the detailed description or claims of the present invention.

### [Description of Drawings]

FIG. 1 is a diagram schematicallyshowing a configuration of the vaccine management system according to one embodiment of the present invention.
FIG.2 is a diagram showing an example of a subject identification code and information on vaccine for vaccination used in one embodiment of the present invention.
FIG.3 is a diagram showing an example of a vaccine identification code for stock used in one embodiment of the present invention.
FIG.4 is a diagram showing an example of a vaccine registration interface displayed on the vaccine management terminal according to one embodiment of the present invention.
FIGS.5, 6 and 11 are diagrams shoing a flow chart of the method for providing a vaccination service according to one embodiment of the present invention.
FIGS.7 to 10, 12 to 14, and 16 are diagrams showing screens displayed on a display unit of the on-site terminal according to one embodiment of the present invention.
FIG.15 is a diagram showing the concept of post-vaccination management of a vaccinated subject displayed on the user terminal according to one embodiment of the present invention.

### [Best Mode]

Since various modifications may be made to the present invention and the present invention may have various embodiments,specific embodiments will be illustrated in the drawings and described in detail.However, this is not intended to limit the present invention to specific embodiments,and it is to be understood that this includes all modifications, equivalents, and substitutes included in the spirit and technical scope of the present invention.In describing each drawing, similar reference numerals are used for similar components.

The terms "first, ""second," and the like may be used to describe various components, but the components should not be limited by the terms.The terms are used only to distinguish one component from another component. The term "and/or" includes any combination of a plurality of related recited items or any one of a plurality of related recited items.

The terminology used in the present specification is used only to describe particular embodiments and is not intended to limit the present invention.Singular expressions include plural expressions unless the context clearly indicates otherwise.As used herein, the terms such as "comprise, "have," and the like are intended to indicate that there is a feature, number, step, operation, component, part, or combination thereof described in the specification, and it should be understood that the terms do not exclude in advance the possibility of the presence or addition of one or more other features, numbers, steps, operations, components, parts, or combinations thereof.

Unless defined otherwise, all terms used herein, including technical or scientific terms, have the same meaning as commonly understood by those of ordinary skill in the art to which the present invention pertains.The terms such as those defined in the commonly used dictionaries should be construed as having meanings consistent with the meanings in the context of the related art, and are not be construed as ideal or excessively formal meanings unless expressly defined in the present application.

Hereinafter, embodiments according to the present invention will be described in detail with reference to the accompanying drawings.

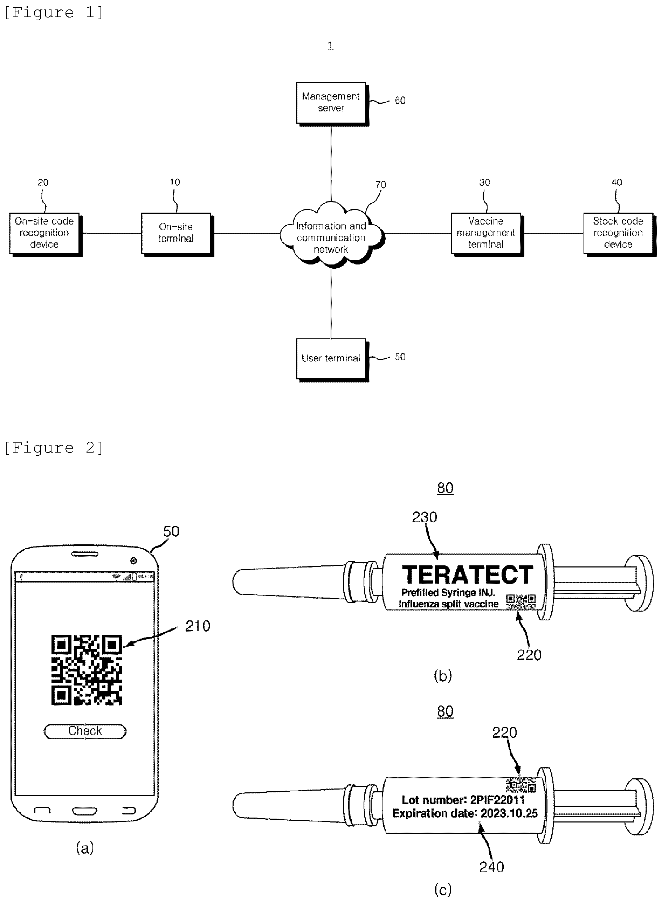

FIG.1 is a diagramschematically showing a configuration of the vaccine management system 1 according to one embodiment of the present invention.

Referring to FIG.1, the vaccine management system 1 according to one embodiment of the present invention may comprise an on-site terminal 10, an on-site vaccination information recognition device 20, a vaccine management terminal 30, a stock code recognition device 40, a user terminal 50, and a management server 60.

The on-site terminal 10 and on-site vaccination information recognition device 20 may be devices installed at the vaccine vaccination site.Wherein the vaccination site may be a location within a medical institution or a location outside the medical institution.

The vaccine management terminal 30 and the vaccine information recognition device for stock 40 may be devices installed in the vaccine storage location of a medical institution where vaccines are stocked and registered at the medical institution.

The user terminal 50 is a terminal held by a subject to be vaccinated or a vaccinated subject, and may be composed of a plurality of terminals.

The management server 60 may generate a subject identification code for each subject to be vaccinated, as described later, and collect and manage the subject identification code and vaccination information identified by the vaccine management terminal 30.Furthermore, the management server 60 may generate and analyze big data based on information on accumulated vaccines used by the vaccine management terminal 30, and accordingly, may predict the optimal vaccine demand for each period/use, and furthermore, may perform automation of vaccine inventory management based on the predicted demand.

Meanwhile, the on-site terminal 10, vaccine management terminal 30, user terminal 50 and management server 60 may be connected to each other through an information and communication network 70.The information and communication network 70 may be implemented as a wired network such as a local area network (LAN), a wide area network (WAN) or a value added network (VAN), or any type of wireless network such as a mobile radio communication network, a satellite communication network, Bluetooth, wireless broadband internet (Wibro) or high speed dDownlink packet access (HSDPA).

Hereinafter, the components of the vaccine management system 1 are described in more detail as follows.

The on-site terminal 10 may be a processor-based device installed at a medical site where a vaccine is vaccinated, and may comprise a camera, a communication unit and a display unit.As an example, the on-site terminal 10 may be various smart devices such as a smartphone, a smart note, a tablet PC, a smart TV and a wearable computer.A vaccination app for administeringa vaccine may be installed and executed on the on-site terminal 10.

The on-site vaccination information recognition device 20 is a device installed at a medical site and may be connected to communicate with the on-site terminal 10 via a wired method or a short-range wireless communication method (e.g., Bluetooth) .That is, the vaccination app may be executed in conjunction with the on-site vaccination information recognition device 20.As an example, the on-site vaccination information recognition device 20 may be an image acquisition device including a camera.

The on-site vaccination information recognition device 20 may recognize the subject identification code, which is a unique identification code of a subject to be vaccinated who visited a medical site, and transmit it to the on-site terminal 10.As an example, as shown in FIG.2A, the on-site vaccination information recognition device 20 may recognize the subject identification code 210 displayed on the display unit of the user terminal 50.As described above, the subject identification code 210 may be generated in the management server 60 and transmitted to the user terminal 50.

In addition, the on-site vaccination information recognition device 20 may recognize vaccination vaccine information printed on the outer surface of a syringe containing the vaccine inside and used for vaccination, or vaccination vaccine information printed on the outer surface of a vial containing the vaccine inside.Hereinafter, embodiments of the present invention will be described assuming only a situation in which thevaccine identification code for vaccination is printed on the syringe.

Information on vaccine for vaccination may include avaccine identification code for vaccination and text information on vaccine for vaccination.Referring to FIG.2B, on one outer surface of the syringe 80, part of the vaccine identification code 220 for vaccination and the drug name 230 of the vaccine for vaccination in the information on vaccine for vaccination may be printed by being attached to a film.Referring to FIG.2C, on the other outer surface of the syringe 80, the remaining part of the vaccine identification code 220 for vaccination and the text information 240 on the vaccine for vaccination including the lot number and expiration date of the vaccine for vaccination in the information on vaccine for vaccination may be printed by being attached to a film.

The vaccine identification code 220 for vaccination may be an identification code that includes information on the drug name of the vaccine for vaccination.The lot number refers to a number assigned to a vaccine manufactured under the same manufacturing conditions and having uniform characteristics and quality, and is also called a "serial number."The expiration date refers to the period during which the vaccine's efficacy or effect may be normally used.

Meanwhile, a particular vaccine manufacturer may manufacture two or more vaccines with different drug names in the same manufacturing facility.In this case, in certain circumstances, vaccines A and B may be assigned the same lot number, and the expiration dates of vaccines A and B may be the same.Therefore, the vaccine management system 1 according to the present invention may prevent a situation in which a subject to be vaccinated with vaccine A is vaccinated with vaccine B,and may efficiently manage a plurality of vaccines, according to the explanation described below.

Referring again to FIG.1, the vaccine management terminal 30 may be a processor-based device installed in a specific location of a medical institution where vaccines are stocked and registered, and may comprise a communication unit and a display unit.As an example, the vaccine management terminal 30 may be a personal computer (PC) or a smart device.The vaccine management terminal 30 may have a vaccine management app installed and executed for stocking vaccines.

The vaccine information recognition device for stock 40 may be a device that recognizes the information on vaccine for stock printed on the vaccine packaging container (e.g., a paper box).The vaccine information recognition device for stock 40 may be connected to communicate with the vaccine management terminal 30 via a wired method or a short-range wireless communication method (e.g., Bluetooth).That is, the vaccine management app may be executed in conjunction with the vaccine information recognition device for stock 40.Thevaccine information recognition device for stock 40 may be an image acquisition device including a camera.

Specifically, the vaccine may be stocked as a unit of a vaccine packaging container, and two or more vaccines (i.e., vaccine vials) may be contained within a vaccine packaging container.As an example, 50 vaccines may be contained in a vaccine packaging container.In this case, two or more vaccines contained in the vaccine packaging container have the same lot number and also the same expiration date.That is, two or more vaccines may be vaccines having uniform characteristics and quality. Therefore, the vaccine identification code for stock printed on the vaccine packaging container may be the vaccine identification code of each of two or more vaccines for stock.

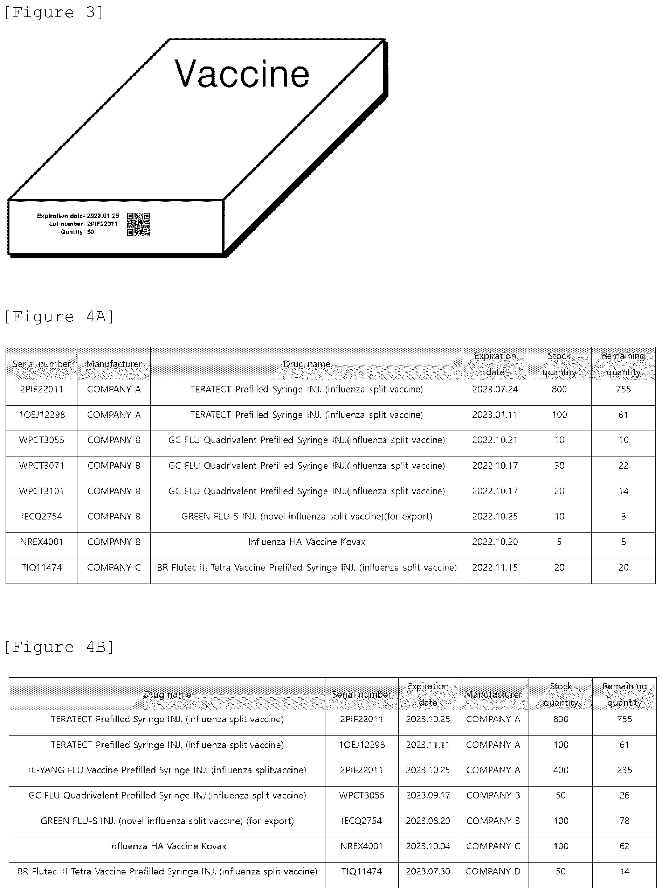

The information on vaccine for stock printed on the vaccine packaging container may include the vaccine identification code for stock, and text information on vaccine for stock, including the lot number, expiration date and stock quantity of the vaccine for stock.As an example, referring to FIG.3, avaccine identification code 310 for stock that includes only information on the drug name and manufacturer of the vaccine for stock may be printed on a vaccine packaging box 300, and text information on vaccine for stock that includes the lot number, expiration date and stock quantity of the vaccine for stock may be printed adjacent to the vaccine identification code 310 for stock.

Again, referring to FIG.1, the vaccine information recognition device for stock 40 may recognize the vaccine identification code for stock printed on the vaccine packaging container and transmit it to the vaccine management terminal 30.In addition, the vaccine information recognition device for stock 40 may recognize the text information on vaccine for stock (i.e., information on expiration date, lot number and stock quantity) printed on the vaccine packaging container and transmit them to the vaccine management terminal 30.The vaccine management terminal 30 may extract the drug name and manufacturer of the vaccine for stock from the received vaccine identification code for stock, and register the vaccine for stock by using the extracted drug name and manufacturer of the vaccine for stock, and information on the expiration date, lot number and stock quantity, which is the additionally received text information on vaccine for stock.

That is, the vaccine management app executed on the vaccine management terminal 30 may display a vaccine registration interface on the display unit of the vaccine management terminal 30 and register the vaccine for stock by entering various information extracted from the vaccine identification code for stock and text information on vaccine for stock into the vaccine registration interface.For this purpose, scanning or optical character recognition (OCR) method may be used.

A variety of vaccines for stock may be registered as described above.In this case, the vaccine management information, which is information on vaccine being registered, may include at least one of the following information: drug name, manufacturer, lot number, expiration date and stock quantity.

A variety of vaccines for stock may be registered as described above.In this case, the vaccine management information, which is information on vaccine being registered, may include at least one of the following information: drug name, manufacturer, lot number, expiration date and stock quantity.

FIG.4 is a diagram showing an example of a vaccine registration interface displayed on a vaccine management terminal 30.

Referring to FIG.4A, the vaccine management terminal 30 may store vaccine management information for each lot number based on the lot number of vaccine.As described above, since vaccines with the same lot number have uniform characteristics and quality, the vaccine management terminal 30 may store vaccine management information for each lot number to facilitate vaccine management and vaccine tracking in the event of a vaccination accident.That is, the drug name, manufacturer, expiration date, stock quantity, remaining quantity, and the like of stocked and registered vaccines may be managed for each lot number.That is, vaccine management information may be managed for each lot number.

Referring to FIG.4B, the drug name, lot number, expiration date, manufacturer, stock quantity, remaining quantity, and the like may be managed for each of stocked and registered vaccines.In particular, in FIG.4, there are TERATECT Prefilled Syringe INJ. (influenzasplit vaccine) and IL-YANG FLU Vaccine Prefilled Syringe INJ. (influenzasplit vaccine), which are two vaccines with the same lot number and expiration date.

Referring again to FIG.1, the user terminal 50 is a terminal held by a subject to be vaccinated or a vaccinated subject, and may may include a communication unit and a display unit.As an example, the user terminal 50 may be various smart devices such as a smartphone.A user app for vaccination and self-diagnosis may be installed and executed on the user terminal 50.Through the user app, identity information for issuing a subject identification code may be transmitted to the management server 60, self-diagnosis questionnaire information may be displayed from the management server 60, self-diagnosis status information corresponding to the self-diagnosis questionnaire information may be transmitted to the management server 60, and other post-vaccination management may be performed in conjunction with the management server 60.

Hereinafter, the method for operating a vaccine management system 1, i.e., the method for providing a vaccination service, will be described in more detail.

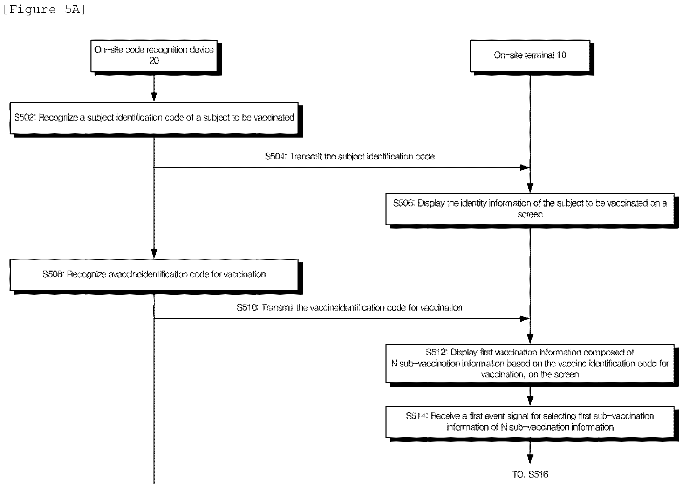

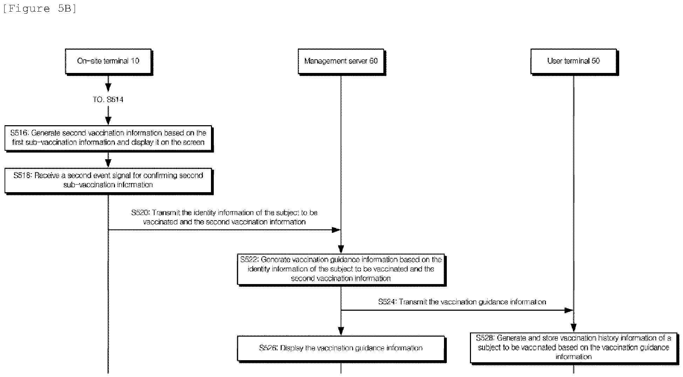

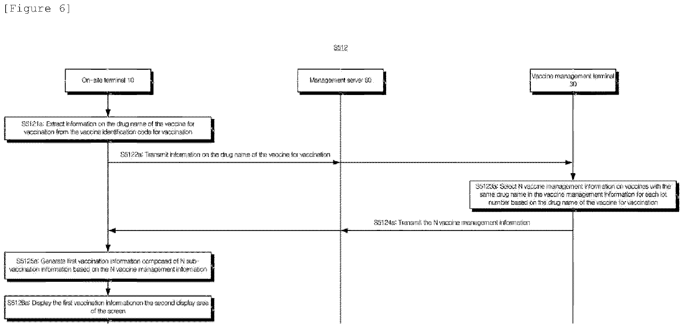

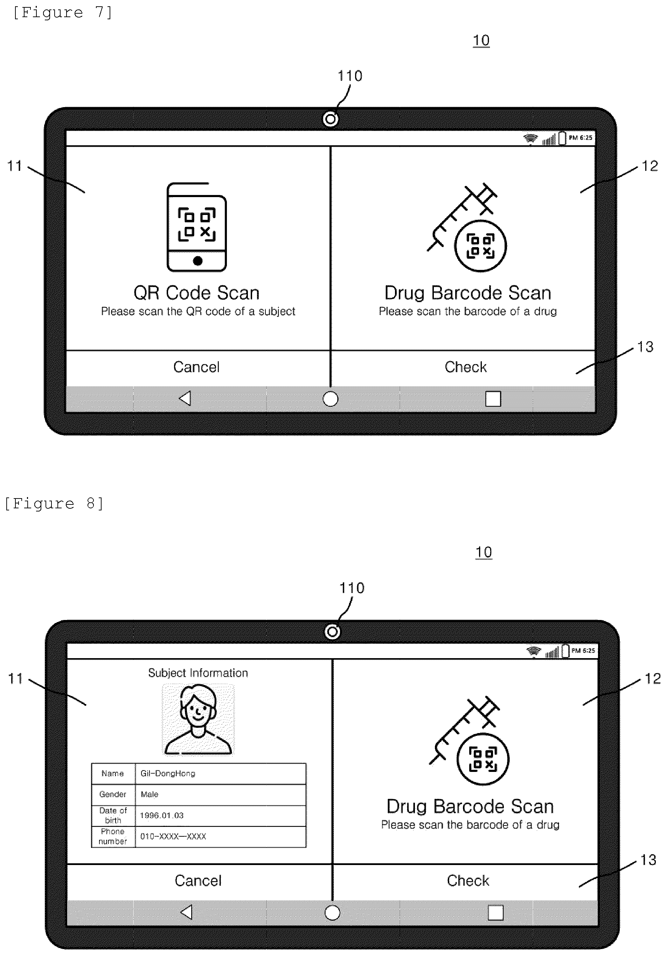

FIGS.5 and 6are diagrams showing a flow chart of the method for providing a vaccination service according to one embodiment of the present invention, and FIGS. 7 to 10 are diagrams showing screens displayed on a display unit of the on-site terminal 10 according to one embodiment of the present invention.In this case, the screens of FIGS.7 to 10 may be execution screens of the vaccination app of the on-site terminal 10.

Before explaining the steps shown in FIGS.5 and 6, the initial screen of the vaccination app of the on-site terminal 10 is described as follows.

Referring to FIG.7, an on-site terminal 10 may have a camera 110 installed on the side where the display unit is provided.The camera 110 may capture images of the vaccination site, and in particular, may capture images of the vaccination site where the subject to be vaccinated and/or medical staff are located.

In addition, the execution screen of the vaccination app may display various information to the subject to be vaccinated and medical staff.In this case, the screen of the vaccination app may include a first display area 11 and a second display area 12 aligned left and right.

The first display area 11 may be an area that displays information related to a subject identification code, and the second display area 12 may be an area that displays information related to avaccine identification code for vaccination.As an example, the first display area 11 may be placed on the left side on the execution screen of the vaccination app, and the second display area 12 may be placed on the right side on the execution screen of the vaccination app.However, the present invention is not limited thereto, and the first display area 11 may be placed on the right side of the execution screen on the vaccination app, and the second display area 12 may be placed on the left side on the execution screen of the vaccination app.A detailed explanation of this will be given later.

Hereinafter, referring first to FIG.5, the process performed at each step will be described in detail.

In step S502, the on-site vaccination information recognition device 20 may recognize the subject identification code of the subject to be vaccinated displayed on the user terminal 50.

In step S504, the on-site vaccination information recognition device 20 may transmit the recognized subject identification code to the on-site terminal 10.

In step S506, the on-site terminal 10 may display the identity information of the subject to be vaccinated on the screen of the display unit of the on-site terminal 10 based on the subject identification code.

Specifically, referring to FIG.8,when the on-site terminal 10 receives a subject identification code from the on-site vaccination information recognition device 20, it may display the identity information of the subject to be vaccinated (i.e.,"information on the vaccinated subject" of FIG.8) on the first display area 11 of the screen.In this case, the on-site terminal 10 may be operated in conjunction with the management server 60, the identity information of the subject to be vaccinated may be registered in advance in the management server 60, and the on-site terminal 10 may receive the identity information of the subject to be vaccinated from the management server 60 and display it on the first display area 11 of the screen.Wherein the identity information of the subject to be vaccinated may include information on the name, gender, date of birth and phone number of the subject to be vaccinated.

Referring again to FIG.5, in step S508, the on-site vaccination information recognition device 20 may recognize information on vaccine for vaccination printed on a syringe containing the vaccine for vaccination.Wherein the information on vaccine for vaccination may be a vaccine identification code for vaccination or text information on vaccine for vaccination.As described above, the text information on vaccine for vaccination may be composed of information on the lot number and expiration date of the vaccine for vaccination (see FIG.2).

In step S510, the on-site vaccination information recognition device 20 may transmit the recognized information on vaccine for vaccination to the on-site terminal 10.

In step S512, the on-site terminal 10 may display first vaccination information composed of N sub-vaccination information based on the information on vaccine for vaccination, on the screen of the display unit of the on-site terminal 10.

Hereinafter, referring to FIG.6, and the like, the detailed steps of step S512 will be described.

FIG.6 is a diagram showing a flow chart of detailed steps of one embodiment of step S512.Wherein it is assumed that the vaccine identification code for vaccination in the information on vaccine for vaccination is recognized.

In step S5121a, the on-site terminal 10 may extract information on the drug name of the vaccine for vaccination from thevaccine identification code for vaccination, and in step S5122a, the on-site terminal 10 may transmit information on the drug name of the vaccine for vaccination to the vaccine management terminal 30 through the management server 60.

In this case, it is assumed that the vaccine management terminal 30 stores vaccine management information for each lot number (see FIG.4A).Therefore, in step S5123a, the vaccine management terminal 30 may select N vaccine management information on vaccines with the same drug name in the vaccine management information for each lot number based on the drug name of the vaccine for vaccination received from the on-site terminal 10, and in step S5124a, the vaccine management terminal 30 may transmit the N vaccine management information to the on-site terminal 10.

Thereafter, in step S5125a, the on-site terminal 10 may generate first vaccination information composed of N sub-vaccination information based on the N vaccine management information, and in step S5126a, the on-site terminal 10 may display the first vaccination information on the second display area 12 of the screen.

Wherein each of the N sub-vaccination information may have a corresponding relationship with the N vaccine management information.In particular, the sub-vaccination information may be configured to include only a part of the vaccine management information.

According to embodiments, the sub-vaccination information may be any one of two types.The two types may be sub-vaccination information A and sub-vaccination information B.

The sub-vaccination information A may correspond to a vaccine for stock whose expiration date has expired.That is, when the expiration date of a specific vaccine for stock is earlier than the current day, the sub-vaccination information corresponding to the specific vaccine for stock may be sub-vaccination information A.

The sub-vaccination information B may correspond to a vaccine for stock whose expiration date has not expired.That is,when the expiration date of a specific vaccine for stock is later than the current day, the sub-vaccination information corresponding to the specific vaccine for stock may be sub-vaccination information B.

According to embodiments, the sub-vaccination information A may include the lot number of vaccine for stock and a notification message notifying that the expiration date of the vaccine for stock has expired.

According to embodiments, the sub-vaccination information B may include the lot number and remaining quantity of the vaccine for stock.In addition, the sub-vaccination information B may further include the expiration date of the vaccine for stock.

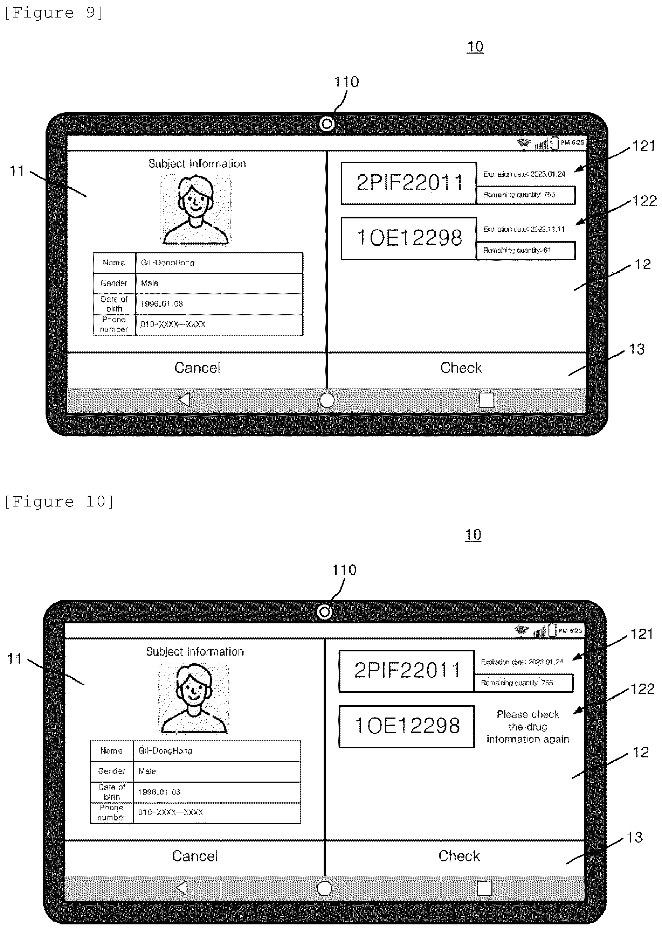

FIGS.9 and 10 are diagrams showing an example in which first vaccination information is displayed on the second display area 12 of the screen.

FIG.9 is a diagram showing an example in which both sub-vaccination information 121, 122 included in the first vaccination information are of the type of sub-vaccination information B.Referring to FIG.9, two sub-vaccination information 121, 122 on vaccines with the same drug name may be aligned vertically and displayed on the second display area 12 of the screen.In this case, as described above, each of the two sub-vaccination information 121, 122 may include the lot number, remaining quantity and expiration date.In particular, information on the lot number, which distinguishes one vaccine from another vaccine, may be displayed in a larger size and emphasized than information on the remaining quantity and expiration date.Therefore, subjects to be vaccinated and medical staff may easily distinguish lot numbers.Meanwhile, the expiration date may be omitted from each of the two sub-vaccination information 121, 122.

FIG.10 is a diagram showing an example in which in the two sub-vaccination information included in the first vaccination information, one sub-vaccination information 121 is of the type of sub-vaccination information B, and another sub-vaccination information 122 is of the type of sub-vaccination information A.Referring to FIG.10, one sub-vaccination information 121 may include the lot number, remaining quantity and expiration date, and another sub-vaccination information 122 may include the lot number and a notification message.

In short, the on-site terminal 10 may compare the expiration date of the vaccines for stock included in the N vaccine management information with the current day to determine the type of the N sub-vaccination information corresponding to the N vaccine management information.In particular, the on-site terminal 10 may prevent a situation in which a vaccine for stock whose expiration date has expired is vaccinated to a subject to be vaccinated by displaying a notification message notifying that the expiration date of the vaccine for stock has expired, on the second display area 12.

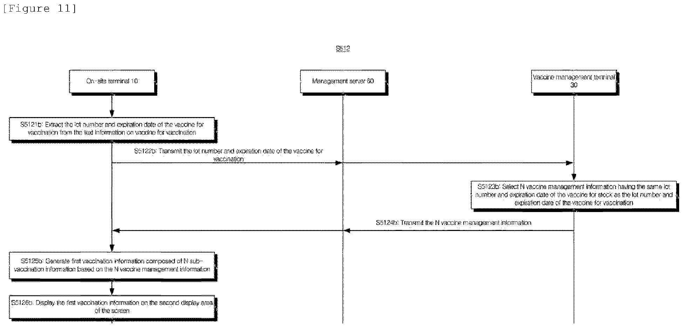

FIG.11 is a diagram showing a flow chart of detailed steps of another embodiment of step S512.Here, it is assumed that text information on vaccine for vaccination in the information on vaccine for vaccination is recognized.

In step S5121b, the on-site terminal 10 may extract the lot number and expiration date of the vaccine for vaccination from the text information on vaccine for vaccination.As an example, the on-site terminal 10 may extract the lot number and expiration date of the vaccine for vaccination from the text information on vaccine for vaccination using an optical character recognition (OCR) module.

In step S5122b, the on-site terminal 10 may transmit the lot number and expiration date of the extracted vaccine for vaccination to the vaccine management terminal 30 through the management server 60.

In this case, it is assumed that the vaccine management terminal 30 stores vaccine management information on a plurality of vaccines as in FIG.4B. Therefore, in step S5123b, the vaccine management terminal 30 may select N vaccine management information having the same lot number and expiration date of the vaccine for stock as the lot number and expiration date of the vaccine for vaccination received from the on-site terminal 10 in the vaccine management information, and in step S5124b, the vaccine management terminal 30 may transmit the N vaccine management information to the on-site terminal 10.

Thereafter, in step S5125b, the on-site terminal 10 may generate first vaccination information composed of N sub-vaccination information based on the N vaccine management information, and in step S5126b, the on-site terminal 10 may display the first vaccination information on the second display area 12 of the screen.

Wherein each of the N sub-vaccination information may have a corresponding relationship with the N vaccine management information.In particular, the sub-vaccination information may be configured to include only a part of the vaccine management information.As an example, the sub-vaccination information may include information on the drug name and lot number in the information included in the vaccine management information.As another example, the sub-vaccination information may include information on the drug name and lot number in the information included in the vaccine management information, and may further include information on the expiration date and remaining quantity.

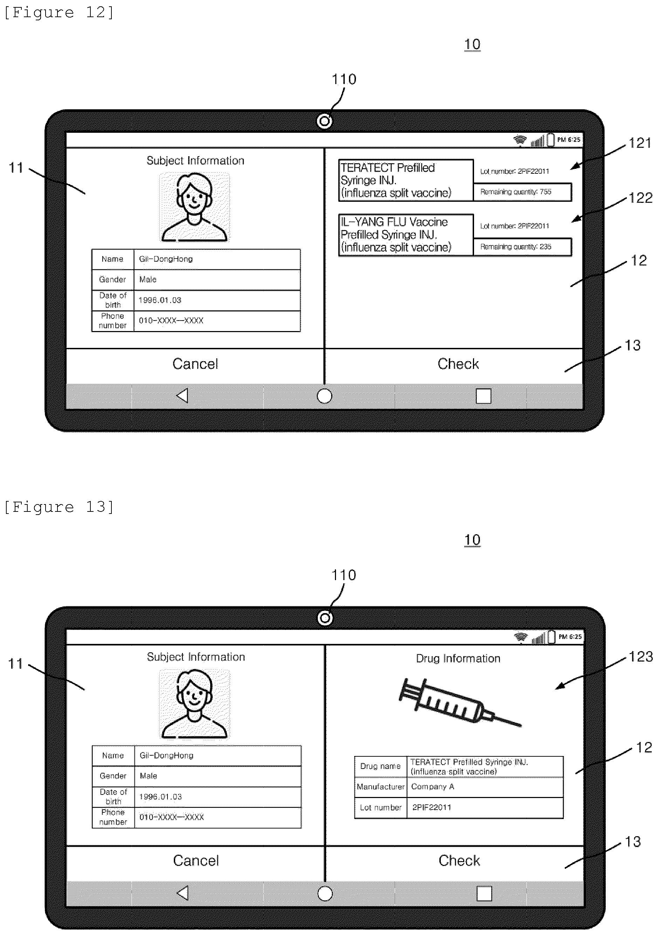

Referring to FIG.12, two sub-vaccination information 121, 122 having the same lot number and expiration date but different drug names may be aligned vertically and displayed on the second display area 12 of the screen.In this case, as described above, each of the two sub-vaccination information 121, 122 may include information on the drug name, lot number and remaining quantity, and information on the expiration date may be omitted.In particular, the drug name that distinguishes one vaccine from another vaccine may be displayed in a larger size and emphasized than information on the lot number and remaining quantity.Therefore, subjects to be vaccinated and medical staff may easily distinguish between the drug names of different vaccines.Meanwhile, although not shown in FIG.12, each of the two sub-vaccination information 121, 122 may further include information on the expiration date along with information on the drug name,lot number and remaining quantity.

Referring again to FIG.5, in step S514, the on-site terminal 10 may receive a first event signal for selecting first sub-vaccination information of N sub-vaccination information.

In this case, the first event signal may be a touch event signal in which the medical staff touches the display unit of the on-site terminal 10.In addition, as shown in FIG.2, the drug name 230, lot number and expiration date 240 of the vaccine for vaccination may be printed on the film of the syringe 80.

According to embodiments,when the recognized information on vaccine for vaccination is a vaccine identification code for vaccination, the medical staff may input the first event signal by comparing the lot number 230 of the vaccine for vaccination printed on the syringe 80 with the N sub-vaccination vaccination information.Hereinafter, it is assumed that the first sub-vaccination information 121 having the same lot number as the lot number 230 of the vaccine for vaccination in the two sub-vaccination information 121, 122 shown in FIG.9 is selected.

According to another embodiment,when the recognized information on vaccine for vaccination is text information on vaccine for vaccination, the medical staff may input the first event signal by comparing the drug name 230, lot number and expiration date 240 of the vaccine for vaccination printed on the syringe 80 with the N sub-vaccination information.Hereinafter, it is assumed that the first sub-vaccination information 121 having the same drug name of the vaccine for stock as the drug name 230 of the vaccine for vaccination in the two sub-vaccination information 121, 122 shown in FIG.12 is selected.

In step S516, the on-site terminal 10 may generate second vaccination information based on the first sub-vaccination information and display it on the screen of the display unit of the on-site terminal 10.

Specifically, when the first sub-vaccination information is selected, the on-site terminal 10 may select first vaccine management information corresponding to the first sub-vaccination information of the N vaccine management information, and may generate the second vaccination information including the drug name, manufacturer and lot number of vaccine for stock in the first vaccine management information and display them on the second display area 12 of the screen.That is, the second vaccination information may be information that reconfirms the first sub-vaccination information selected by the medical staff.FIG.13 is a diagram showing an example in which second vaccination information 123 is displayed on the second display area 12 of the screen.Meanwhile, although not shown in FIG.13, the second vaccination information may further include the expiration date of the vaccine for stock, along with the drug name, manufacturer and lot number of vaccine for stock.

Referring again to FIG.5, in step S518, the on-site terminal 10 may receive a second event signal for confirming second sub-vaccination information.

In this case, the second event signal may also be a touch event signal in which the medical staff touches the display unit of the on-site terminal 10.As an example, the medical staff may input the second event signal into the on-site terminal 10 by confirming the identity information of the subject to be vaccinated and the second vaccination information with the subject to be vaccinated, and then clicking the confirmation button 13.

In step S520, the on-site terminal 10 may transmit the identity information of the subject to be vaccinated and the second vaccination information to the management server 60.

In step S522, the management server 60 may generate vaccination guidance information based on the identity information of the subject to be vaccinated and the second vaccination information.In this case, the vaccination guidance information may include the identity information of the subject to be vaccinated, the vaccination date and time, the vaccination location, and the drug name, manufacturer and lot number of vaccine for vaccination.Wherein the vaccination date and time may correspond to the input time of the second event signal, and the vaccination location may correspond to the location of the on-site terminal 10.

In step S524, the management server 60 may transmit the vaccination guidance information to the user terminal 50 of the subject to be vaccinated, and in step S526,the user terminal 50 may display the vaccination guidance information on the display unit.

In addition, in step S528, the management server 60 may generate and store vaccination history information of a subject to be vaccinated (i.e., a vaccinated subject) based on the vaccination guidance information.In this case, steps S502 to S528 may be performed for all of a plurality of subjects to be vaccinated, and the management server 60 may store the vaccination history information of the plurality of vaccinated subjects for each lot number, similarly as described above.

According to embodiments, when all of steps S502 to S528 are performed, the medical staff may administer a vaccine for vaccination having a confirmed lot number to the subject to be vaccinated.

Meanwhile, although not shown in FIG.5, after step S518, the on-site terminal 10 may transmit a command to reduce the remaining quantity in the first vaccine management information corresponding to the vaccine for vaccination to the vaccine management terminal 30 through the management server 60.In this case, the vaccine management terminal 30 may reduce the remaining quantity in the first vaccine management information by "1" based on the received command to reduce the remaining quantity.That is, when the vaccine for vaccination is vaccinated to a subject to be vaccinated, the remaining quantity in the first vaccine management information stored in the vaccine management terminal 30 may be reduced by "1."Accordingly, the remaining quantity in the first vaccine management information corresponding to the vaccine for vaccination may be automatically managed without the intervention of medical staff.

In short, the method for providing a vaccination service according to one embodiment of the present invention may display check information that must be confirmed for vaccination of a subject to be vaccinated on the screen of an on-site terminal 10 based on a subject identification code and information on vaccine for vaccination (vaccine identification code or vaccine text information) recognized by an on-site vaccination information recognition device 20.In this case, the screen of the on-site terminal 10 may include first and second display areas 11, 12 that are displayed simultaneously, wherein the first display area 11 may display the identity information of the subject to be vaccinated corresponding to the subject identification code of the check information, and the second display area 12 may display the vaccination information corresponding to the information on vaccine for vaccination of the check information.

In this case, the vaccination information may include first and second vaccination information.The first vaccination information may include N sub-vaccination information having the same drug name but different lot numbers.The second vaccination information may be vaccine management information related to the first sub-vaccination information having the same lot number as the lot number of vaccine for vaccination printed on the syringe in the N sub-vaccination information.Alternatively, the first vaccination information may include N sub-vaccination information having the same lot number and expiration date but different drug names.The second vaccination information may be vaccine management information related to the first sub-vaccination information having the same drug name of the vaccine for stock as the drug name of the vaccine for vaccination printed on the syringe 80 in the N sub-vaccination information.

Since the identity information of the subject to be vaccinated and the information on vaccine for vaccination are simultaneously displayed on the screen of the on-site terminal 10, the subject to be vaccinated and the medical staff may easily check at the vaccination site whether the vaccine to be vaccinated is the same vaccine that the subject to be vaccinated applied for.Accordingly, misvaccination accidents, in which a subject to be vaccinated receives a vaccine different from the one intended for vaccination, may be prevented in advance.

In particular, there may be vaccines having the same lot number and expiration date but different drug names.That is, a particular vaccine manufacturer manufactures two or more vaccines with different drug names in the same manufacturing facility, and there may be cases where the two or more vaccines have the same lot number and expiration date. In such a situation, medical staff at the vaccination site must check all of the drug name, lot number and expiration date of the vaccine for vaccination before vaccinating the subject to be vaccinated, but they may mistakenly administer the vaccine without checking the above information, resulting in misvaccination.

In addition, in the past, there were many cases where medical staff at the vaccination site performed vaccination by only checking the drug name of the vaccine without checking the lot number.When registering the fact of completion of vaccination, there was a situation where medical staff did not accurately register the lot number of vaccine vaccinated to a subject, but instead registered a random lot number.In this case, even if the drug name is the same, when the lot number is different, it cannot be considered the same vaccine, and therefore, when an adverse event occurs after a subject is vaccinated, it is impossible to find other subjects vaccinated with the same vaccine, which leads to a problem in which vaccination accidents spread.However, according to the present invention, since the lot number is necessarily confirmed at the time of vaccination, the vaccine is vaccinated to the subject to be vaccinated, and the fact of completion of vaccination is automatically registered, misregistration accidents of the vaccine may also be prevented in advance.

In addition, in a situation where the vaccine was vaccinated normally, when registering the fact of completion of vaccination, there was a situation where medical staff did not accurately register the lot number of vaccine vaccinated to a subject, but instead directly registered a different lot number.Even in this case, even if the drug name is the same, when the lot number is different, it cannot be considered the same vaccine, and therefore, when an adverse event occurs after a subject is vaccinated, it is impossible to find other subjects vaccinated with the same vaccine, which leads to a problem in which vaccination accidents spread.However, according to the present invention, since the drug name, lot number and expiration date is necessarily confirmed at the time of vaccination, the vaccine is vaccinated to the subject to be vaccinated, and the fact of completion of vaccination is automatically registered, misvaccination and misregistration accidents of the vaccine may be prevented in advance.

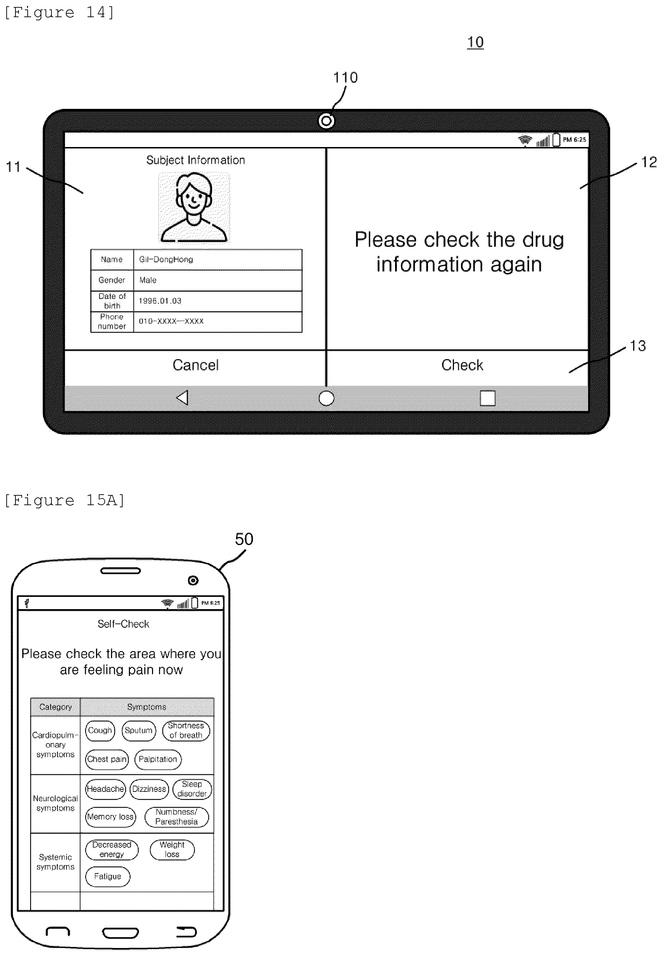

Meanwhile, when the recognized information on vaccine for vaccination is text information on vaccine for vaccination, the on-site terminal 10 may compare the expiration date of the vaccine for stock included in the N vaccine management information with the current day.As described above, the expiration dates of the N vaccine management information may be the same.In this case, when the expiration date of the N vaccine management information is earlier than the current day, the on-site terminal 10 may display a notification message notifying that the expiration date of the vaccine for stock has expired on the second display area 12.An example of a notification message is shown in FIG.14. In this case, steps S512 to S526 described above are not performed.Accordingly, the situation in which a vaccine for stock whose expiration date has expired is vaccinated to a subject to be vaccinated may be prevented.

Meanwhile, the post-vaccination management of the vaccinated subject performed after step S528 is described as follows.In this case, as described above, it is assumed that the management server 60 stores the vaccination history information of the plurality of vaccinated subjects for each lot number.In addition, the contents described below are performed through the conjunction of the plurality of user terminals 50 and the management server 60, but may be performed at individual points in time for each user terminal 50.

First, the management server 60 may transmit self-diagnosis questionnaire information to a plurality of user terminals 50 held by each of a plurality of vaccinated subjects.The self-diagnosis questionnaire information may be confirmed to the vaccinated subject through a user app installed on the user terminal 50.

As an example, the self-diagnosis questionnaire information may include the first self-diagnosis questionnaire information for cardiopulmonary symptoms (including details of "cough, ""sputum, ""shortness of breath, ""chest pain" and "palpitation"), the second self-diagnosis questionnaire information for neurological symptoms (including details of "headache, ""dizziness, ""sleep disorder, ""memory loss" and "numbness/paresthesia"), the third self-diagnosis questionnaire information for systemic symptoms (including details of "decreased energy, ""weight loss" and "fatigue"), the fourth self-diagnosis questionnaire information for digestive symptoms (including details of "heartburn,""abdominalgia/stomachache,""diarrhea, ""nausea/vomi ting, ""dyspepsia" and "flatulence"), the fifth self-diagnosis questionnaire information for mental symptoms (including details of "depression, ""anxiety" and "poor concentration"), the sixth self-diagnosis questionnaire information for otolaryngology symptoms (including details of "hyposmia" and "hypogeusia"), the seventh self-diagnosis questionnaire information for ophthalmic symptoms (including details of "keratoconjunctivitis" and "poor vision"), the eighth self-diagnosis questionnaire information for skin symptoms (including details of "hair loss" and "skin rash"), the ninth self-diagnosis questionnaire information for gynecological symptoms (including details of "menstrual irregularity" and "abnormal vaginal bleeding"), and the tenth self-diagnosis questionnaire information for urinary symptoms (including details of "decreased sexual function" and "abnormal urinary pattern") .This is as shown in FIG.15A.

Next, each of the plurality of user terminals 50 may generate self-diagnosis status information of the vaccinated subject corresponding to the self-diagnosis questionnaire information based on the click event of the vaccinated subject and transmit it to the management server 60.

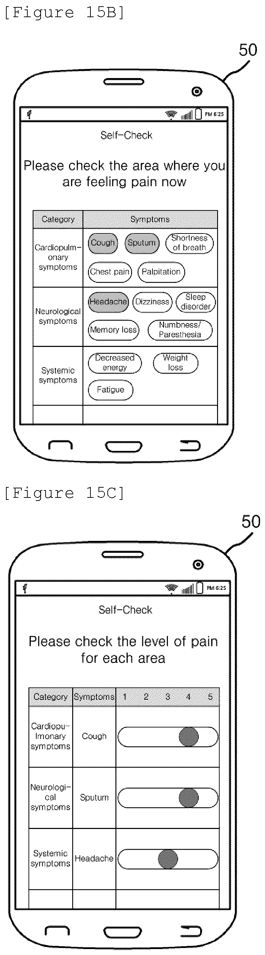

As an example, referring to FIG.15B, the user may select at least one detailed information corresponding to the area of pain in the detailed information for each self-diagnosis questionnaire information displayed on the user terminal 50.Thereafter, referring to FIG.15C, the user terminal 50 may display a pain level input object for each selected detailed information, and the user may input his/her pain level (i.e., any one of levels 1 to 5) through the pain level input object for each selected detailed information.Accordingly, self-diagnosis status information of the vaccinated subject may be generated.

Continuing, the management server 60 may store self-diagnosis status information of each of the plurality of vaccinated subjects for each lot number of vaccine.

Thereafter, the management server 60 may track adverse events of the plurality of vaccinated subjects based on the self-diagnosis status information stored for each lot number of vaccine.

That is, as described above, since vaccines with the same lot number are assumed to be uniform vaccines, the management server 60 may track adverse events of the plurality of vaccinated subjects for each lot number.

The specific details of tracking adverse events of the plurality of vaccinated subjects for each lot number are as follows.In this case, the content of tracking adverse events will be explained based on subjects vaccinated with the vaccine corresponding to the first lot number in the plurality of lot numbers.

First, the management server 60 may calculate the number of vaccinated subjects with adverse events in the plurality of subjects vaccinated with the first vaccine corresponding to the first lot number.

In this case, the vaccinated subjects with adverse events may be defined as the vaccinated subjects for the first vaccine whose level of adverse symptoms exceeded the threshold level.In this case, the management server 50 may set the level of adverse symptoms in various ways based on the level of detailed information for each self-diagnosis questionnaire information. When there is no vaccinated subject with adverse events, the content described below does not apply.

Next, the management server 60 may transmit an adverse event notification message for the vaccinated subject with adverse events to the terminal of the medical institution.Wherein the medical institution may be the medical institution that the vaccinated subject with adverse events visited for the vaccination of the first vaccine.As an example, when adverse events of headache and vomiting were detected in vaccinated subject A, the management server 60 may transmit an adverse event notification message such as "Adverse events of headache and vomiting have been detected in vaccinated subject A, so please check" to the terminal of the medical institution along with the self-diagnosis status information of vaccinated subject A.Accordingly, it is possible to prevent the situation in which the adverse events of the vaccinated subject with adverse events become more severe.

In addition, the management server 60 may determine whether the number of vaccinated subjects with adverse events exceeds a preset threshold number.In other words, the management server 60 may calculate an adverse event rate defined as the ratio of the number of vaccinated subjects with adverse events to the total number of plurality of subjects vaccinated with the first vaccine, and may determine whether the adverse event rate exceeds a preset threshold rate (for example, 2%).

In this case, when the management server 60 determines that the adverse event rate exceeds the threshold rate, the management server 60 may transmit an intensive management notification message to a user terminal of a vaccinated subject with no adverse events who has been vaccinated with the first vaccine but has not experienced any adverse events, and the user terminal of the vaccinated subject with no adverse events may display the intensive management notification message.As an example, the intensive management notification message may be a message such as "An adverse event has been detected for the first vaccine, so please closely check the health status of xxx."Accordingly, according to the present invention, the problem of the spread of vaccination accidents may be further prevented.

Meanwhile, according to another embodiment of the present invention, the management server 60 may collect self-diagnosis status information of a plurality of vaccinated subjects and produce statistics.That is, the vaccine management system 1 according to the present invention may provide statistical analysis data on vaccine side effects to medical staff (vaccination management agency), and thus may implement a centralized vaccine control system such as a status of side effect aggregation and tracking of side effect vaccines.In this case, the real-time side effect aggregation data may be aggregated and provided to the vaccination management agency as statistical figures by gender, age, product, post-vaccination side effect reaction time, type of side effect, and the like through the self-diagnosis described above, and data on the occurrence of side effects in the vaccinated subject, such as reverse tracing and recall of side effect vaccines, may be aggregated.

In particular, the management server 60 may calculate an average pain level by gender and age for each of a plurality of detailed items of self-diagnosis status information.In this case, when the pain level of the subject vaccinated with the first vaccine in the plurality of vaccinated subjects exceeds the average pain level by gender and/or age for the first detailed item in the plurality of detailed items, a pain level exceeding notification message may be transmitted to the user terminal of the subject vaccinated with the first vaccine or the terminal of the medical institution.Through this, the subject vaccinated with the first vaccine may be intensively managed.

Meanwhile, the on-site terminal 10 may be placed on a desk provided at the vaccination site, and the medical staff may perform the vaccination of a subject to be vaccinated at the front side of the on-site terminal 10.In addition, as described in FIGS.7 to 10 and FIGS.12 to 14, the identity information of the subject to be vaccinated may be displayed on the first display area 11 located on the left side of the screen of the vaccination app, and the vaccination information may be displayed on the second display area 12 located on the right side of the screen of the vaccination app.In general, the subject to be vaccinated first confirms his/her identity information and then confirms the vaccination information.

In this case, depending on the structure or route of the vaccination site, the subject to be vaccinated may be located on the left side of the site terminal 10 or on the right side of the site terminal 10.In particular, when the subject to be vaccinated is located on the right side of the on-site terminal 10, the subject to be vaccinated generally first checks the second display area 12 on the right side of the screen of the vaccination app, which is in the same direction as his/her position, wherein the second display area 12 displays vaccination information, and thus, the subject to be vaccinated checks his/her identity information displayed on the first display area 11 and then checks the vaccination information displayed on the second display area 12 again.That is, the direction of the gaze of the subject to be vaccinated with respect to the on-site terminal 10 is "second display area 12 → first display area 11 → second display area 12."This may cause inconvenience to the subject to be vaccinated.

Therefore, according to one embodiment of the present invention, the on-site terminal 10 may set the arrangement positions of the first and second display areas 11, 12 on the screen of the on-site terminal 10 based on at least one of the location of the subject to be vaccinated at the vaccination site and the location of the medical staff administering the vaccine for vaccination at the vaccination site.

Specifically, the screen of the on-site terminal 10 may be divided into a left area and a right area.Meanwhile, in FIGS.7 to 10 and 12 to 14, it was explained that the first display area 11 is displayed on the left area of the screen of the on-site terminal 10, and the second display area 12 is displayed on the right area of the screen of the on-site terminal 10.

In addition, the on-site terminal 10 may capture the vaccination site through a camera 110 to create an image of the vaccination site, and analyze the created image of the vaccination site to calculate the location of the medical staff who will administer the vaccine for vaccination.To this end, the vaccination app may include image analysis tools.

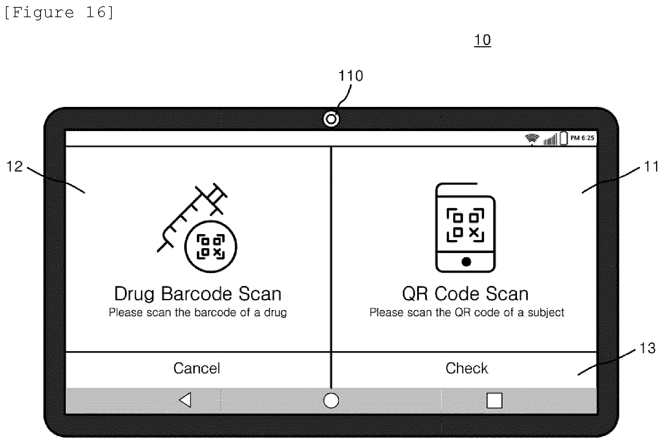

In this case,when the location of the medical staff is on the left side of the on-site terminal 10, the first display area 11 may be placed on the right area of the screen of the on-site terminal 10, and the second display area may be placed on the left area of the screen of the on-site terminal 10, as shown in FIG.16. In addition,when the location of the medical staff is on the right side of the on-site terminal 10, the first display area 11 may be placed on the left area of the screen of the on-site terminal 10, and the second display area 12 may be placed on the right area of the screen of the on-site terminal 10, as described in FIGS.7 to 10 and 12 to 14.

In particular, the medical staff holds the on-site vaccination information identification device 20 for vaccination.This may be a criterion for distinguishing the medical staff and the subject to be vaccinated.That is, the on-site terminal 10 may extract the shape of the on-site vaccination information identification device 20 from the image of the vaccination site, and may correspond the location of the shape of the on-site vaccination information identification device 20 to the location of the medical staff.Meanwhile, extracting the shape of the on-site vaccination information identification device 20 may be performed based on a template matching algorithm.

Specifically, when the extracted shape of the on-site vaccination information recognition device 20 is located in the left area of the image of the vaccination site, the on-site terminal 10 may determine that the location of the medical staff is on the left of the on-site terminal 10.In addition,when the shape of the on-site vaccination information recognition device 20 is located in the right area of the image of the vaccination site, the on-site terminal 10 may determine that the location of the medical staff is on the right of the on-site terminal 10.

In addition, embodiments of the present invention may be embodied in the form of program commands, which may be executed through various computer means and recorded on a computer-readable medium.The computer readable medium may include program instructions, data files, data structures, and the like, alone or in combination. The program commands recorded on the media may be those specially designed and configured for the present invention or may be those that are well known and available to one of ordinary skill in the computer software art.Examples of computer-readable recording media include a magnetic media such as a hard disk, a floppy disks, and a magnetic tape, an optical media such as CD-ROM and DVD, magneto-optical media such as a floptical disk, and a hardware device specifically configured to store and execute program instructions, such as ROM, RAM, a flash memory, and the like.Examples of program instructions include not only machine language codes such as those generated by a compiler, but also high-level language codes that can be executed by a computer using an interpreter, or the like.The hardware device as described above may be configured to operate as one or more software modules to perform the operations of embodiments of the present invention, and vice versa.

As described above, the present invention has been described by specific matters such as specific components,limited embodiments and drawings, but these have been provided only to help the overall understanding of the present invention, and the present invention is not limited to the above embodiments, and those with common knowledge in the art to which the present invention pertains can make various modifications and variations from this description. Therefore, the spirit of the present invention should not be limited to the described embodiments, and all things equivalent to the claims or having equivalent modifications thereof as well as the claims described below are included in the scope of the spirit of the present invention.

## Claims

1. A vaccine management system, comprising:
an on-site vaccination information recognition device for recognizing a subject identification code of a subject to be vaccinated and information on vaccine for vaccination printed on a syringe or vial; and
an on-site terminal for displaying check information that must be confirmed for vaccination of the subject to be vaccinated based on the subject identification code and information on vaccine for vaccination transmitted from the on-site vaccination information recognition device, on a screen,
wherein the screen of the on-site terminal includes: a first display area for displaying the identity information of the subject to be vaccinated corresponding to the subject identification code of the check information; and a second display area for displaying the vaccination information corresponding to the information on vaccine for vaccination of the check information.

2. The vaccine management system according to claim 1, further comprising:
a vaccine information recognition device for stock that is received at a medical institution for recognizing a vaccine identification code for stock and text information on vaccine for stock printed on a packaging container of the vaccine for stock; and
a vaccine management terminal for extracting at least one of the drug name, lot number, manufacturer, expiration date and stock quantity of the vaccine for stock from the vaccine identification code for stock and text information on vaccine for stock transmitted from the vaccine information recognition device for stock and storing vaccine management information,
wherein the vaccine management terminal stores the vaccine management information for each of a plurality of vaccines for stock.

3. The vaccine management system according to claim 2,
wherein the information on vaccine for vaccination corresponds to the vaccine identification code for vaccination,
wherein the vaccine management terminal stores vaccine management information for each lot number based on the lot number of vaccine, and
wherein each of the vaccine management information for each lot number further includes at least one of the drug name, manufacturer, expiration date, stock quantity and remaining quantity, along with the lot number of vaccine for stock.

4. The vaccine management system according to claim 3,
wherein the on-site terminal extracts the drug name of the vaccine for vaccination based on thevaccine identification code for vaccination transmitted from the on-site vaccination information recognition device and transmits it to the vaccine management terminal,
wherein the vaccine management terminal selects N (an integer of 1 or more) vaccine management information of the vaccine management information for each lot number based on the drug name of the vaccine for vaccination and transmits them to the on-site terminal, and
wherein the on-site terminal displays the vaccination information on the second display area based on the N vaccine management information.

5. The vaccine management system according to claim 4,
wherein the on-site terminal displays first vaccination information composed of N sub-vaccination information of the vaccination information on the second display area,
wherein each of the N sub-vaccination information includes the lot number and the expiration date of the corresponding vaccine management information of the N vaccine management information, and
wherein the lot number of the sub-vaccination information is displayed with more emphasis than the expiration date of the sub-vaccination information on the second display area.

6. The vaccine management system according to claim 2,
wherein the information on vaccine for vaccination corresponds to text information on vaccine for vaccination,
wherein the vaccine management terminal stores the vaccine management information for each of a plurality of vaccines for stock, including the vaccines for stock,
wherein the on-site terminal extracts the lot number and expiration date of the vaccine for vaccination from the text information on vaccine for vaccination transmitted from the on-site vaccination information recognition device and transmits them to the vaccine management terminal,
wherein the vaccine management terminal selects N (an integer of 1 or more) vaccine management information having the same lot number and expiration date of the vaccine for stock as the lot number and expiration date of the vaccine for vaccination in the vaccine management information for each of the plurality of vaccines for stock and transmits them to the on-site terminal, and
wherein the on-site terminal displays the vaccination information on the second display area based on the N vaccine management information.

7. The vaccine management system according to claim 6,
wherein the on-site terminal displays first vaccination information composed of N sub-vaccination information of the vaccination information on the second display area,
wherein each of the N sub-vaccination information includes the drug name and the lot number of the corresponding vaccine management information of the N vaccine management information, and
wherein the drug name of the sub-vaccination information is displayed with more emphasis than the lot number of the sub-vaccination informationon the second display area.

8. The vaccine management system according to claim 5 or 7,
wherein, when a first event signal for selecting first sub-vaccination information of the N sub-vaccination information is input, the on-site terminal generates second vaccination information of the vaccination information based on the first sub-vaccination information and displays it on the second display area, and
wherein the second vaccination information includes the drug name, manufacturer and lot number of the first vaccine management information corresponding to the first sub-vaccination information of the N vaccine management information.

9. The vaccine management system according to claim 8,
wherein each of the N sub-vaccination information further includes at least one of the expiration date and remaining quantity of the corresponding vaccine management information of the N vaccine management information, and
wherein the second vaccination information further includes the expiration date of the first vaccine management information corresponding to the first sub-vaccination information of the N vaccine management information.

10. The vaccine management system according to claim 8,
wherein,when a second event signal for confirming the second vaccination information is input, the on-site terminal transmits a command to reduce the remaining quantity in the first vaccine management information to the vaccine management terminal, and
wherein the vaccine management terminal reduces the remaining quantity in the first vaccine management information by "1" based on the command to reduce the remaining quantity.

11. The vaccine management system according to claim 8, further comprising:
a user terminal held by the subject to be vaccinated; and
a management server connected to communicate with the on-site terminal and the user terminal,
wherein, when a second event signal for confirming the second vaccination information is input, the on-site terminal transmits the identity information of the subject to be vaccinated and the second vaccination information to the management server,
wherein the management server generates vaccination guidance information based on the identity information of the subject to be vaccinated and the second vaccination information, and transmits the vaccination guidance information to the user terminal,
wherein the user terminal displays the vaccination guidance information, and
wherein the management server generates and stores vaccination history information of the subject to be vaccinated based on the vaccination guidance information.

12. The vaccine management system according to claim 4 or 6, wherein, when the expiration date of the N vaccine management information has expired, the on-site terminal displays a notification message notifying that the expiration date has expired on the second display area.

13. The vaccine management system according to claim 4 or 6,
wherein the vaccination information includes first vaccination information composed of N sub-vaccination information,
wherein each of the N sub-vaccination information is any one of sub-vaccination information A corresponding to the vaccine for stock whose expiration date has expired and sub-vaccination information B corresponding to the vaccine for stock whose expiration date has not expired, and
wherein the sub-vaccination information A further includes the lot number or drug name of the vaccine for stock included in the corresponding vaccine management information, and further includes a notification message notifying that the expiration date of the vaccine has expired.

14. The vaccine management system according to claim 1, further comprising:
a management server for storing vaccination history information of a plurality of vaccinated subjects for each lot number of vaccine,
wherein the management server transmits self-diagnosis questionnaire information to a plurality of user terminals held by each of the plurality of vaccinated subjects, receives self-diagnosis status information corresponding to the self-diagnosis questionnaire information from each of the plurality of user terminals, and stores the self-diagnosis status information of the plurality of vaccinated subjects for each lot number, and
wherein the management server tracks adverse events of the plurality of vaccinated subjects based on the self-diagnosis status information stored for each lot number.

15. The vaccine management system according to claim 14,
wherein, for each subject vaccinated with a vaccine corresponding to a first lot number, the management server:
calculates a vaccinated subject with adverse events having self-diagnosis status information corresponding to a preset threshold level, and transmits an adverse event notification message for the vaccinated subject with adverse events to a terminal of a medical institution visited by the vaccinated subject with adverse events, and
when the number of the vaccinated subject with adverse events exceeds a preset threshold number,transmits an intensive management notification message for a vaccinated subject with no adverse events who has been vaccinated with the vaccine corresponding to the first lot number but has not experienced any adverse events to a user terminal of the vaccinated subject with no adverse events.

16. The vaccine management system according to claim 14,
wherein the management servercalculates an average pain level by gender and age for each of a plurality of detailed items of self-diagnosis status information received from the plurality of vaccinated subjects, and
when the pain level of the subject vaccinated with the first vaccine of the plurality of vaccinated subjects exceeds the average pain level by any one of gender and age for a first detailed item of the plurality of detailed items, transmits a pain level exceeding notification message to the user terminal of the subject vaccinated with a first vaccine or the terminal of the medical institution.

17. The vaccine management system according to claim 1, wherein the on-site terminal sets the arrangement positions of the first and second display areas on the screen of the on-site terminal based on at least one of the location of the subject to be vaccinated at the vaccination site and the location of the medical staff administering the vaccine for vaccination at the vaccination site.

18. The vaccine management system according to claim 17,
wherein the screen of the on-site terminal is divided into a left area and a right area,
wherein the on-site terminal is equipped with a camera and analyzes the image of the vaccination site captured by the camera to calculate the location of the medical staff, wherein:
when the location of the medical staff is on the left side of the on-site terminal, the first display area is placed on the right area on the screen of the on-site terminal, and the second display area is placed on the left area on the screen of the on-site terminal, and
when the location of the medical staff is on the right side of the on-site terminal, the first display area is placed on the left area on the screen of the on-site terminal, and the second display area is placed on the right area on the screen of the on-site terminal.

19. The vaccine management system according to claim 18,
wherein the on-site terminal extracts the shape of the on-site vaccination information recognition device from the image of the vaccination site, wherein:
when the shape of the on-site vaccination information recognition device is located in the left area of the image of the vaccination site, it is determined that the location of the medical staff is on the left side of the on-site terminal, and
when the shape of the on-site vaccination information recognition device is located in the right area of the image of the vaccination site, it is determined that the location of the medical staff is on the right side of the on-site terminal.

20. A method for providing a vaccination service performed in a vaccine management system comprising an on-site vaccination information recognition device and an on-site terminal connected to communication, comprising the steps of:
recognizing a subject identification code of a subject to be vaccinated and information on vaccine for vaccination printed on a syringe or vial by the on-site vaccination information recognition device and transmitting them to the on-site terminal; and
displaying check information that must be confirmed for vaccination of the subject to be vaccinated based on the subject identification code and information on vaccine for vaccination, on a screen of the on-site terminal,
wherein the screen of the on-site terminal includes: a first display area for displaying the identity information of the subject to be vaccinated corresponding to the subject identification code of the check information; and a second display area for displaying the vaccination information corresponding to the information on vaccine for vaccination of the check information.
